# EUROPEAN PATENT SPECIFICATION

(11) **EP 4 014 949 B1**
(45) Date of publication and mention of the grant of the patent: **31.07.2024**
(21) Application number: 21155464.7
(22) Date of filing: 05.02.2021
(51) Int. Cl.: A61K 6/813, A61K 6/818, A61K 6/804, A61K 6/822, A61C 13/00, A61C 13/08, A61C 13/083

(54) **METHOD FOR PRODUCING A CERAMIC MULTILAYER BLANK**
VERFAHREN ZUR HERSTELLUNG EINES KERAMISCHEN MEHRSCHICHTROHLINGS
PROCÉDÉ DE FABRICATION D'UNE ÉBAUCHE MULTICOUCHE EN CÉRAMIQUE

(30) Priority: 15.12.2020 US 202063125456 P
(43) Date of publication of application: 22.06.2022
(73) Proprietor: DENTSPLY SIRONA Inc., York, PA 17401 (US); DeguDent GmbH, 63457 Hanau (DE)
(72) Inventor: Fecher, Stefan, 63867 Johannesberg (DE); Völkl, Lothar, 63773 Goldbach (DE); Ammon, Daniel, Webster, NY 14580 (US)
(74) Representative: Pichova, Vanda

(56) References cited:
- EP-A1- 3 542 753
- EP-A1- 3 698 752
- WO-A1-2016/071301
- WO-A1-2019/035467
- JP-A- 2019 163 246
- US-A1- 2021 128 283
- DURAN P ET AL: "PREPARATION, SINTERING, AND PROPERTIES OF TRANSLUCENT ER2O3-DOPED TETRAGONAL ZIRCONIA", JOURNAL OF THE AMERICAN CERAMIC SOCIETY, BLACKWELL PUBLISHING, MALDEN, MA, US, vol. 72, no. 11, 1 November 1989 (1989-11-01), pages 2088 - 2093, XP000103093, ISSN: 0002-7820, DOI: 10.1111/J.1151-2916.1989.TB06036.X

## Description

### Field of the Invention

The present invention relates to a method for producing a ceramic multilayer blank comprising at least a first layer of a first ceramic material and at least a second layer of a second ceramic material, wherein the first layer and the second layer are made of ceramic materials of different compositions, which are filled in pourable condition layer-by-layer into a mold and thereafter they are pressed and then sintered.

### Background of the Invention

US 8 936 848 B2 discloses a blank of zirconium dioxide that is used for the preparation of a tooth replacement and comprises a number of layers of different chemical compositions. The individual layers thereby have different percentages of yttrium oxide.

A body of zirconium dioxide exhibits a decrease or increase in chromaticity along a straight line in the L*a*b* color space.

A blank of zirconium dioxide for the preparation of dental objects in accordance with WO 2014/062375 A1 has at least two material regions which have different proportions of tetragonal and cubic crystal phases, wherein in one of the regions the ratio is greater than 1 and in the other region the ratio is lower than 1.

EP 2 371 344 A1 relates to a ceramic body which is enriched with a stabilizing agent from the surface to a desired depth.

WO2016/071301 A1 discloses to use erbium oxide as pink colorant in a ceramic multilayered blank.

EP 3698752 A1 discloses a multilayered ceramic blank where the layers are structured with alternating elevations and valleys.

WO2019/035467 A1 discloses a multilayered dental blank with a structured surface of the first layer that is gingiva colored.

Zirconium dioxide is used as a ceramic material to produce dental restorations. A framework can be milled, for example, from a blank of zirconium dioxide and can then be sintered. In the following processing stages, a veneer is applied manually to the framework, wherein at least one incisor material is applied manually and fused. All of these process steps are time-consuming.

Even when many ceramic materials for dental restorations based on a zirconium dioxide system have already been investigated, it has been found very challenging up to the present to produce a specific ceramic base material, which is able to provide a pink color having a high similarity to human gingiva.

The provision of such a pink colored ceramic material and methods for producing them are more and more required by the dental industry for dental restorations, such as for the production of a full or a partial denture, an implant supported superstructure, or an implant supported denture.

### Objective of the present Invention

In view of the prior art, it was thus an object of the present invention to provide a method to produce a pink colored ceramic material for dental restoration applications wherein said pink colored ceramic material shall be as similar as possible to human gingiva.

### Summary of the Invention

These objects and further objects which are not stated explicitly but are immediately derivable or discernible from the connections discussed herein by way of introduction are achieved by a method having all features of claim 1. Appropriate modifications to the inventive method are protected in dependent claims 2 to 14.

The present invention accordingly provides a method for producing a ceramic multilayer blank as defined in claim 1.

It is thus possible in an unforeseeable manner to provide a method to produce a pink colored ceramic material for dental restoration applications wherein said pink colored ceramic material is similar to human gingiva.

### Brief Description of the Figures

For a more complete understanding of the present invention, reference is made to the following Detailed Description of the Invention considered in conjunction with the accompanying figures, in which:
**Fig. 1** exhibits a schematic of an assembly and the process steps that can be carried out with it in accordance with the present invention.
**Fig. 2** exhibits the assembly shown in Fig. 1 b) in greater detail.
**Fig. 3** exhibits a schematic of an alternative method in accordance with the present invention.

### Detailed Description of the Invention

The expression "substantially free" means in the context of the present invention a concentration of less than 0.0005 weight percent, preferably less than 0.0003 weight percent, and more preferably less than 0.0001 weight percent.

In the invention, the ceramic multilayer blank further comprises a plurality of additional layers, wherein each of said additional layers is made of a ceramic material, which has a different composition than the first layer, and which has a different or identical composition than the second layer;
wherein after filling of the second layer of the second ceramic material in pourable condition, surface of the second layer is structured in such a way that the second layer when viewed across its surface differs from region to region in its height, and then as the first of said additional layers, a layer of a ceramic material of said additional layers in pourable condition is filled into the mold;
wherein after filling of the first of said additional layers of a ceramic material of said additional layers in pourable condition, surface of the first of said additional layers is structured in such a way that the first of said additional layers when viewed across its surface differs from region to region in its height, and then as the second of said additional layers, a layer of a ceramic material of said additional layers in pourable condition is filled into the mold;
wherein if the second of said additional layers is not the last additional layer, the aforementioned method step is repeated until all additional layers in pourable condition are filled into the mold.

In one embodiment therein, the surface of the respective layer is structured in such a way that elevations and depressions are provided.

In one embodiment, the ceramic multilayer blank comprises two to five, preferably two to four, more preferably three, additional layers, wherein each of said additional layers is made of a ceramic material, which has a different composition than the first layer, and which has a different or identical composition than the second layer.

In one embodiment, the ceramic material of the second layer and/or the ceramic material of all additional layers comprises at least one oxide of the elements Mn, Co, Fe, Tb, Pr and Er;
and/or wherein the ceramic material of the first layer further comprises at least one oxide of the elements Mn, Co, Fe, Tb and Pr.

In one embodiment, the ceramic material of the first layer and/or the ceramic material of the second layer and/or the ceramic material of the third layer and/or the ceramic material of all additional layers of the ceramic multilayer blank further comprises between 0.0005 and 0.02 wt%, preferably between 0.0005 and 0.01 wt%, and more preferably between 0.0005 and 0.05 wt% of an oxide of the element Mn; and/or
the ceramic material of the first layer and/or the ceramic material of the second layer and/or the ceramic material of the third layer and/or the ceramic material of all additional layers of the ceramic multilayer blank further comprises between 0.0005 and 0.1 wt%, preferably between 0.0005 and 0.07 wt%, and more preferably between 0.0005 and 0.05 wt% of an oxide of the element Co.

In one embodiment, the content of the at least one oxide of the element Mn in the respective ceramic material of the respective layer of the multilayer blank is continuously increasing from the second layer to the third layer; or from the second layer via the first of the additional layers continuously further to the last of the additional layers.

In one embodiment, the content of the at least one oxide of the element Mn is continuously increasing by totally 1 to 50 ppm, preferably by 1 to 30 ppm, and more preferably by 1 to 10 ppm; from the second layer to the third layer; or from the second layer to the last of the additional layers; wherein the ceramic multilayer blank is substantially, preferably completely, free of any oxide of the element Co.

In one embodiment, the content of the at least one oxide of the element Co in the respective ceramic material of the respective layer of the multilayer blank is continuously increasing from the second layer to the third layer; or from the second layer via the first of the additional layers continuously further to the last of the additional layers.

In one embodiment, the content of the at least one oxide of the element Co is continuously increasing by totally 1 to 100 ppm, preferably by 1 to 60 ppm, and more preferably by 1 to 30 ppm; from the second layer to the third layer; or from the second layer to the last of the additional layers; wherein the ceramic multilayer blank is substantially, preferably completely, free of any oxide of the element Mn.

In one embodiment, the content of the oxides of the elements Mn and Co in the respective ceramic material of the respective layer of the multilayer blank is continuously increasing from the second layer to the third layer; or from the second layer via the first of the additional layers continuously further to the last of the additional layers.

In one embodiment, the content of the oxides of the elements Mn and Co is continuously increasing for the oxide of the element Mn by totally 1 to 35 ppm, preferably by 1 to 20 ppm, and more preferably by 1 to 6 ppm; while continuously increasing for the oxide of the element Co by totally 1 to 70 ppm, preferably by 1 to 40 ppm, and more preferably by 1 to 20 ppm;
from the second layer to the third layer; or from the second layer to the last of the additional layers.

In one embodiment, the content of the oxides of the elements Mn and Co in the respective ceramic material of the respective layer of the multilayer blank is continuously increasing from the second layer to the third layer; or from the second layer via the first of the additional layers continuously further to the last of the additional layers; and
the content of the oxides of the elements Fe, Tb, Pr and Er in the respective ceramic material of the respective layer of the multilayer blank is continuously decreasing from the second layer to the third layer; or from the second layer via the first of the additional layers continuously further to the last of the additional layers.

In one embodiment, the ceramic material of all layers of the ceramic multilayer blank comprises zirconium dioxide doped with yttrium oxide (Y2O3), calcium oxide (CaO), magnesium oxide (MgO) and/or cerium oxide (CeO2), wherein the zirconium dioxide is doped with yttrium oxide, wherein the percentage of yttrium oxide in the second, third and/or additional layers is between 1 wt% and 15 wt%, preferably between 2 wt% and 11 wt%, and more preferably between 2.5 wt% and 10 wt%.

In one embodiment, the ceramic material of all layers of the ceramic multilayer blank comprises zirconium dioxide doped with yttrium oxide (Y2O3), calcium oxide (CaO), magnesium oxide (MgO) and/or cerium oxide (CeO2), wherein the zirconium dioxide is doped with yttrium oxide, wherein the percentage of yttrium oxide in the pink colored layer is between 0.3 wt% and 10.5 wt%, preferably between 0.6 wt% and 7.5 wt%, and more preferably between 0.75 wt% and 7 wt%.

The method outlined above defines that after subsequently filling of a first and a second layer, the second layer is provided with a surface structure such that when viewed along its surface comprises regions of different heights , i.e., it does not have a uniform fill height, and a third layer or a first additional layer that differs from the second layer in its composition is then filled into the mold.

As an alternative, an intermediate layer with a different composition compared to the second layer is filled into the mold on top of the second layer and both layers are mixed before the third layer or the first additional layer is then filled into the die. It is in particular provided for the material of the intermediate layer to be mixed with the material of the third layer or the first additional layer starting from the free surface of the intermediate layer over a height, which is twice or approximately twice the height of the intermediate layer. Furthermore, it is in particular provided for the material of the intermediate layer to be the same material as that used for the third layer or the first additional layer.

In accordance with the invention, for the first alternative initially a first and a second layer of material in pourable condition is filled into a mold. The material of the second layer may be a tooth-colored zirconium dioxide granular material that, for example, has a bulk density between 1g/cm³ and 1.4 g/cm³, preferably in the range between 1.15 g/cm³ and 1.35 g/cm³. After filling of the granular material of the second layer, which can have a grain size D50 between 40µm and 70µm, the surface is smoothed, and then to shape or form a structure that has depressions (valleys) and elevations which in particular extend parallel to one another, in particular however, concentric or parallel to one another. For this purpose it is in particular provided that the structure is formed through an element that moves relative to the second layer, in particular rotates relative to the second layer, that in particular with a wave-like, comb-like or saw-tooth-like section structures the second layer in its surface region. There is a quasi "raking" of the second layer surface to form the structure with alternating elevations and valleys.

In particular the structure is to be formed such that the volume of the elevations is equal to, or approximately equal to, that of the depressions or valleys.

The saw-tooth-like element preferably has V-shaped teeth that are symmetrical in shape and has flanks that enclose an angle between 15° and 45°. The distance between neighbouring teeth, i.e., the distance from peak to peak, should be between 1 mm and 4 mm, preferably between 1mm and 3mm.

The pourable third or first additional ceramic material is then filled into the mold, and increases in quantity starting from the troughs that form the valleys of the structure, so that as a consequence, there is a virtually constant increase in the percentage of the third layer or the first additional layer across the height of the elevations. After smoothing of the surface, the layers are compressed to achieve an approximate density in the region of 3 g/cm³. Pre-sintering is then carried out at a temperature between 700°C and 1100°C, in particular in the range between 800°C and 1000°C for a time between, for example, 100 and 150 minutes. The blank so produced is then worked, for example, through milling and/or grinding to yield a desired dental restoration that is then sintered until a final density can be attained that for zirconium dioxide, for example, is in the range 6.0 to 6.1 g/cm³.

Complete/final sintering to full density is carried out, for example, for a time between 10 minutes and 250 minutes at a temperature in the range 1300°C to 1600°C. Complete sintering may also be carried out at a somewhat higher temperature. If sintering is carried out at a temperature that is, for example, 100°C higher than that given by the manufacturer of the starting material, then this is referred to as over-sintering, with a sintering time corresponding to that given for complete sintering.

Complete sintering is in particular carried out in the range 1350°C to 1550°C, wherein densities between 6.0 and 6.1g/cm³, in particular between 6.04 and 6.09 g/cm³ can be achieved.

The penetration of the second and, the third or the additional, layers results in the advantage that different physical and optical properties can be achieved across the height of the blank. Thus, once the second layer is colored to the required extent, a tooth-colored edge region can be obtained after complete sintering, across the transition region formed by the penetrating second and, third layer or first additional layer, materials, in which the intensity (a synonym to this expression "intensity" in the sense of the present invention is the expression "chroma") of the tooth color decreases continuously and at the same time the translucency increases in the desired manner. The dental restoration is then produced from the blank, in particular by milling, taking into account the course of the layer, wherein the dental restoration is "laid" in the blank such that the tooth incisor extends in the region of the third layer or the first additional layer.

Independently thereof, a continuous transition between the second and, the third or the additional, layers is provided on the basis of the teaching of the invention, so that color/translucency decreases or increases continuously and also the bending strength can be adjusted in such a way that the region of the dental restoration, which is subject to an extensive loading, has a higher bending strength than the regions which are not so heavily loaded.

In a preferred manner, the possibility of mixing the second and, the third or the additional, layer materials is provided by rotating an element, in particular, about an axis extending along the longitudinal axis of the mold, in order to achieve the structure, which is also referred to as a wave-like or saw-tooth-like structure, by displacing material of the surface of the layer. There is also the possibility of forming the structure by means of a pressure element which acts on the second layer in the direction of the surface and which has, in particular, elevations extending in its surface with depressions extending between them so that the negative form of the element, also referred to as a stamp, is impressed into the surface of the second layer. Then, as explained above, the ceramic material of the third layer or the first additional layer is filled and then smoothed to press the layers together and then pre-sinter the pressed object.

The invention is also characterized in that the second and, the third layer or the first additional layer, are mutually penetrated in their superposed regions across a height H which is a 1/15 to a quarter, in particular 1/10 to 1/5, of the total height of the second and, third layer or first additional, layers.

The second layer should have a height in an unstructured state which corresponds approximately to 1/2 to 2/3 of the sum of the second and, third layer or first additional, layers.

The invention is characterized, inter alia, by the following measures. First, a first layer of a first ceramic material and a second layer of a second ceramic material are filled in pourable condition layer-by-layer into a mold. Herein, the first layer and the second layer are made of ceramic materials of different compositions; wherein the first layer is a pink colored layer, wherein the first ceramic material comprises 2 to 25 wt%, preferably 4 to 17 wt%, and more preferably 5 to 12 wt%, erbium oxide; and wherein the second layer is a first tooth-colored layer, wherein the ceramic material of the second layer consists predominantly of zirconium dioxide.

The filling height of the second layer corresponds approximately to 1/2 to 2/3 blank height of second and, third or all additional, layers before pressing.

The surface is then structured by a specially structured element or a stamp, wherein the structure can be designed such that there is a continuous transition of the properties from the second material to the second and, third layer or first additional, material. Also, the surface geometry of the second layer can be aligned with the diffusion coefficients of the layer materials.

Preferably, a rotating element is used which is lowered into the mold, i.e., into the mold, in which the second layer is located, and then is immersed into the second layer to the extent required. The surface is selectively structured by rotating the element, which is structured on the layer side like a wave-like or comb-like element. Alternatively, the surface may be structured by a press plunger with a suitable geometry.

Subsequently, the mold is filled with the second and the, third or first additional, ceramic material. The usual pressing of the ceramic materials and pre-sintering then takes place.

There is also no departure from the invention if a material for forming an intermediate layer is then filled into the mold after the filling of the second layer.

The intermediate layer may, for example, have a height of 1/10 to 1/5 of the total height of the second and, the third or the additional, layers to be filled into the mold. The intermediate layer material is then mixed with the second layer. In this case, mixing takes place with an element which at least penetrates into the second layer to a depth which corresponds to the height of the intermediate layer. Then, a layer corresponding to the previously described third or first additional layer is filled into the mold. As explained above, the ceramic materials are then pressed to a blank and pre-sintered to obtain, in particular, a dental restoration from the blank so produced by milling. A further processing step is complete sintering to full density. The material of the intermediate layer should be that of the third or first additional layer.

The above method of the present invention has been found suitable for the production of a full or a partial denture, an implant supported superstructure, or an implant supported denture.

In the sense of the present invention, the inventive method can therefore (as described above) be applied to provide a multilayer blank, which is presintered or full sintered at the end of the inventive method.

The inventive method provides thereby a ceramic multilayer blank, which is used by a skilled person to digitally position a required dental restoration as listed above in the blank, wherein it has to be paid attention to position the future area of the human gingiva completely inside of the first ceramic layer of the blank while the future dentin area shall be positioned entirely in the other layers (second and third ceramic layer; or second and additional layers) of the blank. After having produced the dental restoration out of the blank, the skilled person has still to subsequent work in the transition area of the human gingiva to the dentin area by applying stains or glaze materials before finally glazing the dental restoration.

The present invention thus addresses the problem of provide a method to produce a pink colored ceramic material for dental restoration applications wherein said pink colored ceramic material shall be as similar as possible to human gingiva.

The following non-limiting examples are provided to illustrate an embodiment of the present invention and to facilitate understanding of the invention but are not intended to limit the scope of the invention, which is defined by the claims appended hereto.

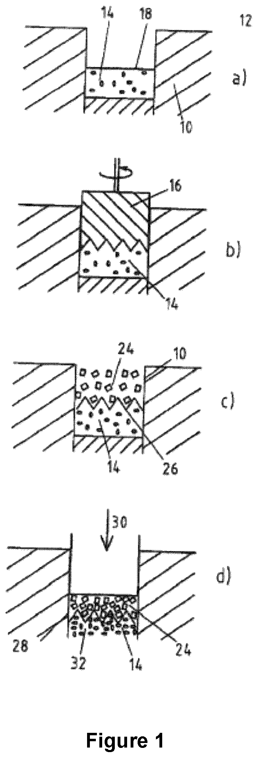

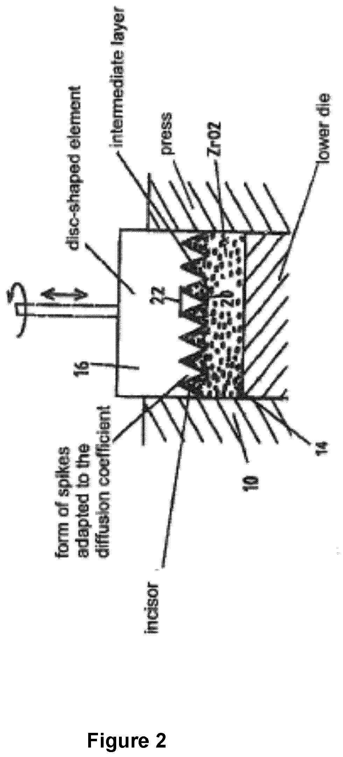

With reference to Figures 1 and 2, the production of a blank from which a corresponding dental restoration can be produced is described.

The provision of a first layer of a first ceramic material is not explicitly illustrated in all Figures even when foreseen in all embodiments of the present invention. Thus, as described before a first layer of a first ceramic material is always filled first in pourable condition into a mold, wherein the first ceramic material comprises 2 to 25 wt%, preferably 4 to 17 wt%, and more preferably 5 to 12 wt%, erbium oxide.

Then, as shown in Fig. 1a) a second material 14 is filled subsequently on the top of the surface of the first layer of the first ceramic material into the mold (other synonym for the expression "mold" is the expression "die") 10 of a press 12.

Subsequently, a third or first additional layer 24 is filled into the die 10 (Fig. 1 c), wherein the total height of the layers 14 and 24 is equal to twice the height of the layer 14 in the unstructured state without restriction of the teaching according to the invention.

If the second layer 14 preferably has a height which corresponds to half the total height H of the second and, the third or first additional layer, 14, 24, then the height of the second layer 14 can also be 1/2 H to 2/3 H and thus that of the third or first additional layer 24 1/3 H to 1/2 H.

The smoothed surface is then structured according to step b). For this purpose, for example, a disc-shaped or plate-shaped or web-shaped element 16 is used, which in the example embodiment has a toothed geometry on the layer side, so that a corresponding negative structure is formed in the surface 18 of the layer 14 by displacing material. This structure is represented by concentrically extending elevations and surrounding valleys. The distance between the elevation (peak) and the valley (depression), i.e., the clear distance between the projection 20 and the valley bottom 22 according to Figure 2, should be approximately 1/5 of the height of all layers.

In particular, it is provided that the structure is formed such that the volume of the elevations is equal to or approximately equal to the volume of the depressions or valleys.

Since the material of the third or first additional layer 24 penetrates to the base of the valleys 26 in the surface 18 of the layer 14, there is a continuous transition between the properties of the layer 14 and the layer 24, after the layers 24, 14 have been pressed according to Fig. 1 d). The transition or intermediate layer is denoted by the reference numeral 28 in Figure 1 d).

The layers 14, 24 are pressed by means of a stamp 30, with a pressure between 1000 bar and 3000 bar.

The pourable material, i.e., in the state in which it is filled into the die 10, has a bulk density between 1 g/cm³ and 1.4 g/cm³. After pressing, the density is approximately between 3.0 and 3.5 g/cm³.

As a result of the structuring, a density of up to 2 g/cm³ is obtained in the transition region between the unmixed regions of the second and the, third or first additional, layers 14, 24 before the layers 14 and 24 are compacted. The transition region can also be referred to as middle layer 28.

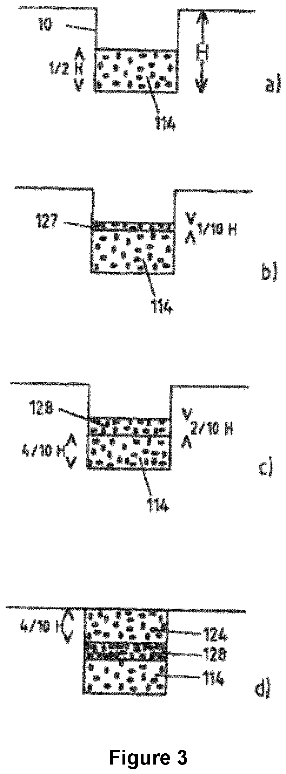

After pressing, the produced blank is ejected from the mold 10 and pre-sintered in the customary manner at a temperature of between 800°C and 1000°C for a period of time between 100 minutes and 150 minutes.
With reference to Fig. 3 an alternative method, which follows the teaching according to the invention, wherein a blank or a dental restoration is to be prepared which provides a largely continuous transition between a second layer and a third or first additional layer.

Also herein, a first layer of a first ceramic material is filled first in pourable condition into a mold, wherein the first ceramic material comprises 2 to 25 wt%, preferably 4 to 17 wt%, and more preferably 5 to 12 wt%, erbium oxide.

Then, according to Figure 3a, a second ceramic material, which corresponds to the layer 14 according to Figure 1, is filled subsequently into a die 10. The corresponding layer in Figure 3a is indicated by the numeral 114. The height of this layer may be half the height of the total layers which are filled into the die 10. A layer 127 with a thickness which in the example embodiment is 1/10 of the total height of the layers is then applied to the layer 114. The material of the layer 127 can correspond to that of the third or first additional layer 24 according to Figure 1. The layer 127 is then mixed with a surface region of the layer 114 over a depth corresponding to the thickness of the layer 127. This forms an intermediate layer 128 having a thickness of 2/10 of the total height of the layers. A further layer 124, which corresponds to the third or first additional layer 24 according to Figure 1, is then applied to the intermediate layer 128. The height of the layer 124 in the example embodiment is thus 4/10 of the total height H. The layers 124, 128, 114 are then pressed together in accordance with the example embodiment of Figure 1 to enable performance of the process steps pre-sintering, working and complete sintering as described. Working can naturally be carried out after complete sintering.

While the principles of the invention have been explained in relation to certain particular embodiments, and are provided for purposes of illustration, it is to be understood that various modifications thereof will become apparent to those skilled in the art upon reading the specification. Therefore, it is to be understood that the invention disclosed herein is intended to cover such modifications as fall within the scope of the appended claims. The scope of the invention is limited only by the scope of the appended claims.

## Claims

1. A method for producing a ceramic multilayer blank for producing a full or a partial denture, an implant supported superstructure or an implant supported denture, comprising at least a first layer of a first ceramic material and at least a second layer of a second ceramic material, wherein the first layer and the second layer are made of ceramic materials of different compositions, which are filled in pourable condition layer-by-layer into a mold and thereafter they are pressed and then sintered, wherein the first layer is a pink colored layer,
wherein the first ceramic material comprises 2 to 25 wt%, preferably 4 to 17 wt%, and more preferably 5 to 12 wt%, erbium oxide,
**characterized in that** the ceramic multilayer blank further comprises a plurality of additional layers, wherein each of said additional layers is made of a ceramic material, which has a different composition than the first layer, and which has a different or identical composition than the second layer;
wherein after filling of the second layer of the second ceramic material in pourable condition, surface of the second layer is structured in such a way that the second layer when viewed across its surface differs from region to region in its height, and then as the first of said additional layers, a layer of a ceramic material of said additional layers in pourable condition is filled into the mold;
wherein after filling of the first of said additional layers of a ceramic material of said additional layers in pourable condition, surface of the first of said additional layers is structured in such a way that the first of said additional layers when viewed across its surface differs from region to region in its height, and then as the second of said additional layers, a layer of a ceramic material of said additional layers in pourable condition is filled into the mold;
wherein if the second of said additional layers is not the last additional layer, the aforementioned method step is repeated until all additional layers in pourable condition are filled into the mold,
wherein the method further comprises a step of digitally positioning the full or the partial denture, the implant supported superstructure or the implant supported denture in the ceramic multilayer blank, wherein it has to be paid attention to positioning the future area of human gingiva completely inside of the first ceramic layer of the blank, while the future dentin area shall be positioned entirely in the other layers of the blank.

2. Method according to claim 1, **characterized in that** the surface of the respective layer is structured in such a way that elevations and depressions are provided.

3. Method according to claim 1 or 2, , **characterized in that** the ceramic multilayer blank comprises two to five, preferably two to four, more preferably three, additional layers, wherein each of said additional layers is made of a ceramic material, which has a different composition than the first layer, and which has a different or identical composition than the second layer.

4. Method according to claim 1, **characterized in that** the ceramic material of the second layer and/or the ceramic material of all additional layers comprises at least one oxide of the elements Mn, Co, Fe, Tb, Pr and Er; and/or wherein the ceramic material of the first layer further comprises at least one oxide of the elements Mn, Co, Fe, Tb and Pr.

5. Method according to claim 4, **characterized in that** the ceramic material of the first layer and/or the ceramic material of the second layer and/or the ceramic material of the third layer and/or the ceramic material of all additional layers of the ceramic multilayer blank further comprises between 0.0005 and 0.02 wt%, preferably between 0.0005 and 0.01 wt%, and more preferably between 0.0005 and 0.05 wt% of an oxide of the element Mn; and/or
the ceramic material of the first layer and/or the ceramic material of the second layer and/or the ceramic material of the third layer and/or the ceramic material of all additional layers of the ceramic multilayer blank further comprises between 0.0005 and 0.1 wt%, preferably between 0.0005 and 0.07 wt%, and more preferably between 0.0005 and 0.05 wt% of an oxide of the element Co.

6. Method according to claim 5 , **characterized in that** the content of the at least one oxide of the element Mn in the respective ceramic material of the respective layer of the multilayer blank is continuously increasing from the second layer to the third layer; or from the second layer via the first of the additional layers continuously further to the last of the additional layers.

7. Method according to claim 6, **characterized in that** the content of the at least one oxide of the element Mn is continuously increasing by totally 1 to 50 ppm, preferably by 1 to 30 ppm, and more preferably by 1 to 10 ppm; from the second layer to the third layer; or from the second layer to the last of the additional layers; wherein concentration of any oxide of the element Co in the ceramic multilayer blank is of less than 0.0005 weight percent.

8. Method according to claim 5, **characterized in that** the content of the at least one oxide of the element Co in the respective ceramic material of the respective layer of the multilayer blank is continuously increasing from the second layer to the third layer; or from the second layer via the first of the additional layers continuously further to the last of the additional layers.

9. Method according to claim 8, **characterized in that** the content of the at least one oxide of the element Co is continuously increasing by totally 1 to 100 ppm, preferably by 1 to 60 ppm, and more preferably by 1 to 30 ppm; from the second layer to the third layer; or from the second layer to the last of the additional layers; wherein concentration of any oxide of the element Mn in the ceramic multilayer blank is of less than 0.0005 weight percent.

10. Method according to claim 5, **characterized in that** the content of the oxides of the elements Mn and Co in the respective ceramic material of the respective layer of the multilayer blank is continuously increasing from the second layer to the third layer; or from the second layer via the first of the additional layers continuously further to the last of the additional layers.

11. Method according to claim 10, **characterized in that** the content of the oxides of the elements Mn and Co is continuously increasing for the oxide of the element Mn by totally 1 to 35 ppm, preferably by 1 to 20 ppm, and more preferably by 1 to 6 ppm; while continuously increasing for the oxide of the element Co by totally 1 to 70 ppm, preferably by 1 to 40 ppm, and more preferably by 1 to 20 ppm;
from the second layer to the third layer; or from the second layer to the last of the additional layers.

12. Method according to claim 5, **characterized in that** the content of the oxides of the elements Mn and Co in the respective ceramic material of the respective layer of the multilayer blank is continuously increasing from the second layer to the third layer; or from the second layer via the first of the additional layers continuously further to the last of the additional layers; and
the content of the oxides of the elements Fe, Tb, Pr and Er in the respective ceramic material of the respective layer of the multilayer blank is continuously decreasing from the second layer to the third layer; or from the second layer via the first of the additional layers continuously further to the last of the additional layers.

13. Method according to one of the preceding claims, **characterized in that** the ceramic material of all layers of the ceramic multilayer blank comprises zirconium dioxide doped with yttrium oxide (Y2O3), calcium oxide (CaO), magnesium oxide (MgO) and/or cerium oxide (CeO2), wherein the zirconium dioxide is doped with yttrium oxide, wherein the percentage of yttrium oxide in the second, third and/or additional layers is between 1 wt% and 15 wt%, preferably between 2 wt% and 11 wt%, and more preferably between 2.5 wt% and 10 wt%.

14. Method according to one of the preceding claims, **characterized in that** the ceramic material of all layers of the ceramic multilayer blank comprises zirconium dioxide doped with yttrium oxide (Y2O3), calcium oxide (CaO), magnesium oxide (MgO) and/or cerium oxide (CeO2), wherein the zirconium dioxide is doped with yttrium oxide, wherein the percentage of yttrium oxide in the pink colored layer is between 0.3 wt% and 10.5 wt%, preferably between 0.6 wt% and 7.5 wt%, and more preferably between 0.75 wt% and 7 wt%.

## Patentansprüche

1. Verfahren zur Herstellung eines keramischen Mehrschichtrohlings zur Herstellung einer Voll- oder Teilprothese, eines implantatgestützten Aufbaus oder einer implantatgestützten Prothese, umfassend mindestens eine erste Schicht aus einem ersten keramischen Material und mindestens eine zweite Schicht aus einem zweiten keramischen Material, wobei die erste Schicht und die zweite Schicht aus keramischen Materialien unterschiedlicher Zusammensetzungen bestehen, die in schüttfähigem Zustand schichtweise in eine Form gefüllt und danach gepresst und anschließend gesintert werden, wobei
die erste Schicht eine pinkfarbene Schicht ist,
wobei das erste keramische Material 2 bis 25 Gew.-%, bevorzugt 4 bis 17 Gew.-% und mehr bevorzugt 5 bis 12 Gew.-% Erbiumoxid umfasst,
**dadurch gekennzeichnet, dass** der keramische Mehrschichtrohling weiter eine Vielzahl von zusätzlichen Schichten umfasst, wobei jede der genannten zusätzlichen Schichten aus einem keramischen Material besteht, das eine andere Zusammensetzung als die erste Schicht hat und das eine andere oder identische Zusammensetzung wie die zweite Schicht hat;
wobei nach dem Einfüllen der zweiten Schicht des zweiten keramischen Materials in schüttfähigem Zustand die Oberfläche der zweiten Schicht so strukturiert wird, dass die zweite Schicht, wenn man über ihre Oberfläche blickt, sich von Bereich zu Bereich in ihrer Höhe unterscheidet, und dann als die erste der genannten zusätzlichen Schichten eine Schicht eines keramischen Materials der genannten zusätzlichen Schichten in schüttfähigem Zustand in die Form gefüllt wird;
wobei nach dem Einfüllen der ersten der genannten zusätzlichen Schichten aus einem keramischen Material der genannten zusätzlichen Schichten in schüttfähigem Zustand die Oberfläche der ersten der genannten zusätzlichen Schichten so strukturiert wird, dass sich die erste der genannten zusätzlichen Schichten, wenn man sie über ihre Oberfläche betrachtet, von Bereich zu Bereich in ihrer Höhe unterscheidet, und dann als zweite der genannten zusätzlichen Schichten eine Schicht aus einem keramischen Material der genannten zusätzlichen Schichten in schüttfähigem Zustand in die Form gefüllt wird;
wobei, wenn die zweite der genannten zusätzlichen Schichten nicht die letzte zusätzliche Schicht ist, der genannte Verfahrensschritt wiederholt wird, bis alle zusätzlichen Schichten in schüttfähigem Zustand in die Form gefüllt sind,
wobei das Verfahren weiter einen Schritt der digitalen Positionierung der Voll- oder Teilprothese, des implantatgestützten Aufbaus oder der implantatgestützten Prothese in dem keramischen Mehrschichtrohling umfasst, wobei darauf zu achten ist, dass der zukünftige Bereich der menschlichen Gingiva vollständig innerhalb der ersten keramischen Schicht des Rohlings positioniert wird, während der zukünftige Dentinbereich vollständig in den anderen Schichten des Rohlings positioniert werden soll.

2. Verfahren nach Anspruch 1, **dadurch gekennzeichnet, dass** die Oberfläche der jeweiligen Schicht so strukturiert wird, dass Erhebungen und Vertiefungen vorgesehen sind.

3. Verfahren nach Anspruch 1 oder 2, **dadurch gekennzeichnet, dass** der keramische Mehrschichtrohling zwei bis fünf, bevorzugt zwei bis vier, mehr bevorzugt drei, zusätzliche Schichten umfasst, wobei jede der genannten zusätzlichen Schichten aus einem keramischen Material besteht, das eine andere Zusammensetzung hat als die erste Schicht und das eine andere oder identische Zusammensetzung hat wie die zweite Schicht.

4. Verfahren nach Anspruch 1, **dadurch gekennzeichnet, dass** das keramische Material der zweiten Schicht und/oder das keramische Material aller zusätzlichen Schichten mindestens ein Oxid der Elemente Mn, Co, Fe, Tb, Pr und Er umfasst; und/oder wobei das keramische Material der ersten Schicht weiter mindestens ein Oxid der Elemente Mn, Co, Fe, Tb und Pr umfasst.

5. Verfahren nach Anspruch 4, **dadurch gekennzeichnet, dass** das keramische Material der ersten Schicht und/oder das keramische Material der zweiten Schicht und/oder das keramische Material der dritten Schicht und/oder das keramische Material aller zusätzlichen Schichten des keramischen Mehrschichtrohlings weiter zwischen 0,0005 und 0,02 Gew.-%, bevorzugt zwischen 0,0005 und 0,01 Gew.-% und mehr bevorzugt zwischen 0,0005 und 0,05 Gew.-% eines Oxids des Elements Mn umfasst; und/oder
das keramische Material der ersten Schicht und/oder das keramische Material der zweiten Schicht und/oder das keramische Material der dritten Schicht und/oder das keramische Material aller zusätzlichen Schichten des keramischen Mehrschichtrohlings weiter zwischen 0,0005 und 0,1 Gew.-%, bevorzugt zwischen 0,0005 und 0,07 Gew.% und mehr bevorzugt zwischen 0,0005 und 0,05 Gew.-% eines Oxids des Elements Co umfasst.

6. Verfahren nach Anspruch 5, **dadurch gekennzeichnet, dass** der Gehalt des mindestens einen Oxids des Elements Mn in dem jeweiligen keramischen Material der jeweiligen Schicht des Mehrschichtrohlings von der zweiten Schicht zur dritten Schicht; oder von der zweiten Schicht über die erste der zusätzlichen Schichten kontinuierlich weiter zur letzten der zusätzlichen Schichten kontinuierlich ansteigt.

7. Verfahren nach Anspruch 6, **dadurch gekennzeichnet, dass** der Gehalt des mindestens einen Oxids des Elements Mn von der zweiten Schicht zur dritten Schicht; oder von der zweiten Schicht zur letzten der zusätzlichen Schichten kontinuierlich um insgesamt 1 bis 50 ppm, bevorzugt um 1 bis 30 ppm und mehr bevorzugt um 1 bis 10 ppm ansteigt; wobei die Konzentration eines Oxids des Elements Co in dem keramischen Mehrschichtrohling geringer als 0,0005 Gew.-% ist.

8. Verfahren nach Anspruch 5, **dadurch gekennzeichnet, dass** der Gehalt des mindestens einen Oxids des Elements Co in dem jeweiligen keramischen Material der jeweiligen Schicht des Mehrschichtrohlings von der zweiten Schicht zur dritten Schicht; oder von der zweiten Schicht über die erste der zusätzlichen Schichten kontinuierlich weiter zur letzten der zusätzlichen Schichten kontinuierlich ansteigt.

9. Verfahren nach Anspruch 8, **dadurch gekennzeichnet, dass** der Gehalt des mindestens einen Oxids des Elements Co von der zweiten Schicht zur dritten Schicht; oder von der zweiten Schicht zur letzten der zusätzlichen Schichten kontinuierlich um insgesamt 1 bis 100 ppm, bevorzugt um 1 bis 60 ppm und mehr bevorzugt um 1 bis 30 ppm ansteigt; wobei die Konzentration jedes Oxids des Elements Mn in dem keramischen Mehrschichtrohling geringer als 0,0005 Gew.-% ist.

10. Verfahren nach Anspruch 5, **dadurch gekennzeichnet, dass** der Gehalt der Oxide der Elemente Mn und Co im jeweiligen keramischen Material der jeweiligen Schicht des Mehrschichtrohlings von der zweiten Schicht zur dritten Schicht; oder von der zweiten Schicht über die erste der zusätzlichen Schichten kontinuierlich weiter zur letzten der zusätzlichen Schichten kontinuierlich ansteigt.

11. Verfahren nach Anspruch 10, **dadurch gekennzeichnet, dass** der Gehalt der Oxide der Elemente Mn und Co für das Oxid des Elements Mn von der zweiten Schicht bis zur dritten Schicht; oder von der zweiten Schicht bis zur letzten der zusätzlichen Schichten kontinuierlich um insgesamt 1 bis 35 ppm, bevorzugt um 1 bis 20 ppm und mehr bevorzugt um 1 bis 6 ppm ansteigt; während er für das Oxid des Elements Co kontinuierlich um insgesamt 1 bis 70 ppm, bevorzugt um 1 bis 40 ppm und mehr bevorzugt um 1 bis 20 ppm ansteigt.

12. Verfahren nach Anspruch 5, **dadurch gekennzeichnet, dass** der Gehalt der Oxide der Elemente Mn und Co im jeweiligen keramischen Material der jeweiligen Schicht des Mehrschichtrohlings von der zweiten Schicht zur dritten Schicht; oder von der zweiten Schicht über die erste der zusätzlichen Schichten kontinuierlich weiter zur letzten der zusätzlichen Schichten kontinuierlich ansteigt; und
der Gehalt der Oxide der Elemente Fe, Tb, Pr und Er im jeweiligen keramischen Material der jeweiligen Schicht des Mehrschichtrohlings von der zweiten Schicht zur dritten Schicht; oder von der zweiten Schicht über die erste der zusätzlichen Schichten kontinuierlich weiter zur letzten der zusätzlichen Schichten kontinuierlich abnimmt.

13. Verfahren nach einem der vorhergehenden Ansprüche, **dadurch gekennzeichnet, dass** das keramische Material aller Schichten des keramischen Mehrschichtrohlings mit Yttriumoxid (Y2O3), Calciumoxid (CaO), Magnesiumoxid (MgO) und/oder Ceroxid (CeO2) dotiertes Zirkoniumdioxid umfasst, wobei das Zirkoniumdioxid mit Yttriumoxid dotiert ist, wobei der prozentuale Anteil an Yttriumoxid in der zweiten, dritten und/oder den zusätzlichen Schichten zwischen 1 Gew.-% und 15 Gew.-%, bevorzugt zwischen 2 Gew.-% und 11 Gew.-% und mehr bevorzugt zwischen 2,5 Gew.-% und 10 Gew.-% ist.

14. Verfahren nach einem der vorhergehenden Ansprüche, **dadurch gekennzeichnet, dass** das keramische Material aller Schichten des keramischen Mehrschichtrohlings Zirkoniumdioxid umfasst, das mit Yttriumoxid (Y2O3), Calciumoxid (CaO), Magnesiumoxid (MgO) und/oder Ceroxid (CeO2) dotiert ist, wobei das Zirkoniumdioxid mit Yttriumoxid dotiert ist, wobei der prozentuale Anteil an Yttriumoxid in der pinkfarbenen Schicht zwischen 0,3 Gew.-% und 10,5 Gew.-%, bevorzugt zwischen 0,6 Gew.-% und 7,5 Gew.-% und mehr bevorzugt zwischen 0,75 Gew.-% und 7 Gew.-% ist.

## Revendications

1. Procédé de fabrication d'une ébauche céramique multicouche pour la fabrication d'une prothèse dentaire complète ou partielle, d'une superstructure implanto-portée ou d'une prothèse dentaire sur implants, comprenant au moins une première couche d'un premier matériau céramique et au moins une seconde couche d'un second matériau céramique, la première et la seconde couche étant constituées de matériaux céramiques de différentes compositions, lesquels sont versés à l'état fluide couche par couche dans un moule et sont ensuite pressés et frittés, dans lequel
la première couche est une couche de couleur rose,
dans lequel le premier matériau céramique comprend de 2 à 25 % en poids, de préférence de 4 à 17 % en poids, et de façon encore préférentielle de 5 à 12 % en poids d'oxyde d'erbium,
**caractérisé en ce que** l'ébauche céramique multicouche comprend en outre plusieurs couches supplémentaires, chacune de ces couches supplémentaires étant constituée d'un matériau céramique dont la composition est différente de celle de la première couche, et dont la composition est différente ou identique à celle de la seconde couche ; dans lequel, après le versement de la seconde couche du second matériau céramique à l'état fluide, la surface de la seconde couche est composée de telle façon que la seconde couche, lorsqu'elle est observée sur toute sa surface, présente des variations de hauteur d'une zone à l'autre, puis en tant que première couche de ces couches supplémentaires, une couche d'un matériau céramique de ces couches supplémentaires est versée dans le moule à l'état fluide ;
dans lequel, après le versement de la première de ces couches supplémentaires d'un matériau céramique de ces couches supplémentaires à l'état fluide, la surface de la première de ces couches supplémentaires est composée de telle façon que la première de ces couches supplémentaires, lorsqu'elle est observée sur toute sa surface, présente des variations de hauteur d'une zone à l'autre, puis en tant que seconde couche de ces couches supplémentaires, une couche d'un matériau céramique de ces couches supplémentaires est versée dans le moule à l'état fluide ;
dans lequel, si la seconde de ces couches supplémentaires n'est pas la dernière, l'étape du procédé susmentionné est répétée jusqu'à ce que toutes les couches supplémentaires à l'état fluide soient versées dans le moule,
dans lequel le procédé comprend en outre une étape de positionnement numérique de la prothèse dentaire complète ou partielle, de la superstructure implanto-portée ou de la prothèse dentaire sur implants dans l'ébauche céramique multicouche, en veillant à positionner la future zone de gencive humaine entièrement à l'intérieur de la première couche céramique de l'ébauche, tandis que la future zone de dentine doit être entièrement positionnée dans les autres couches de l'ébauche.

2. Procédé selon la revendication 1, **caractérisé en ce que** la surface de la couche respective est composée de manière à former des reliefs et des dépressions.

3. Procédé selon la revendication 1 ou 2, **caractérisé en ce que** l'ébauche céramique multicouche comprend de deux à cinq, de préférence de deux à quatre, de façon encore préférentielle trois, couches supplémentaires, chacune de ces couches supplémentaires étant constituée d'un matériau céramique dont la composition est différente de celle de la première couche, et dont la composition est différente ou identique à celle de la seconde couche.

4. Procédé selon la revendication 1, **caractérisé en ce que** le matériau céramique de la seconde couche et/ou le matériau céramique de toutes les couches supplémentaires comprend au moins un oxyde parmi les éléments Mn, Co, Fe, Tb, Pr et Er ; et/ou dans lequel le matériau céramique de la première couche comprend en outre au moins un oxyde parmi les éléments Mn, Co, Fe, Tb et Pr.

5. Procédé selon la revendication 4, **caractérisé en ce que** le matériau céramique de la première couche et/ou le matériau céramique de la seconde couche et/ou le matériau céramique de la troisième couche et/ou le matériau céramique de toutes les couches supplémentaires de l'ébauche céramique multicouche comprend en outre entre 0,0005 et 0,02 % en poids, de préférence entre 0,0005 et 0.01 % en poids, et de façon encore préférentielle entre 0,0005 et 0,05 % en poids d'un oxyde de l'élément Mn ; et/ou
le matériau céramique de la première couche et/ou le matériau céramique de la seconde couche et/ou le matériau céramique de la troisième couche et/ou le matériau céramique de toutes les couches supplémentaires de l'ébauche céramique multicouche comprend en outre entre 0,0005 et 0,1 % en poids, de préférence entre 0,0005 et 0,07 % en poids, et de façon encore préférentielle entre 0,0005 et 0,05 % en poids d'un oxyde de l'élément Co.

6. Procédé selon la revendication 5, **caractérisé en ce que** la teneur en au moins un oxyde de l'élément Mn dans le matériau céramique respectif de la couche respective de l'ébauche multicouche augmente continuellement de la seconde à la troisième couche ; ou de la seconde à la dernière des couches supplémentaires en passant par la première des couches supplémentaires.

7. Procédé selon la revendication 6, **caractérisé en ce que** la teneur en au moins un oxyde de l'élément Mn augmente continuellement de 1 à 50 ppm au total, de préférence de 1 à 30 ppm, et de façon encore préférentielle de 1 à 10 ppm ; de la seconde à la troisième couche ; ou de la seconde à la dernière des couches supplémentaires ; la concentration de tout oxyde de l'élément Co dans l'ébauche céramique multicouche étant inférieure à 0,0005 pour cent en poids.

8. Procédé selon la revendication 5, **caractérisé en ce que** la teneur en au moins un oxyde de l'élément Co dans le matériau céramique respectif de la couche respective de l'ébauche multicouche augmente continuellement de la seconde à la troisième couche ; ou de la seconde à la dernière des couches supplémentaires en passant par la première des couches supplémentaires.

9. Procédé selon la revendication 8, **caractérisé en ce que** la teneur en au moins un oxyde de l'élément Co augmente continuellement de 1 à 100 ppm au total, de préférence de 1 à 60 ppm, et de façon encore préférentielle de 1 à 30 ppm ; de la seconde à la troisième couche ; ou de la seconde à la dernière des couches supplémentaires ; la concentration de tout oxyde de l'élément Mn dans l'ébauche céramique multicouche étant inférieure à 0,0005 pour cent en poids.

10. Procédé selon la revendication 5, **caractérisé en ce que** la teneur en oxydes des éléments Mn et Co dans le matériau céramique respectif de la couche respective de l'ébauche multicouche augmente continuellement de la seconde à la troisième couche ; ou de la seconde à la dernière des couches supplémentaires en passant par la première des couches supplémentaires.

11. Procédé selon la revendication 10, **caractérisé en ce que** la teneur en oxydes des élément Mn et Co augmente continuellement pour l'oxyde de l'élément Mn de 1 à 35 ppm au total, de préférence de 1 à 20 ppm, et de façon encore préférentielle de 1 à 6 ppm ; tandis qu'elle augmente continuellement pour l'oxyde de l'élément Co de 1 à 70 ppm au total, de préférence de 1 à 40 ppm, et de façon encore préférentielle de 1 à 20 ppm ; de la seconde à la troisième couche ; ou de la seconde à la dernière des couches supplémentaires.

12. Procédé selon la revendication 5, **caractérisé en ce que** la teneur en oxydes des éléments Mn et Co dans le matériau céramique respectif de la couche respective de l'ébauche multicouche augmente continuellement de la seconde à la troisième couche ; ou de la seconde à la dernière des couches supplémentaires en passant par la première des couches supplémentaires ; et
la teneur en oxydes des éléments Fe, Tb, Pr et Er dans le matériau céramique respectif de la couche respective de l'ébauche multicouche diminue continuellement de la seconde à la troisième couche ; ou de la seconde à la dernière des couches supplémentaires en passant par la première des couches supplémentaires.

13. Procédé selon l'une des précédentes revendications, **caractérisé en ce que** le matériau céramique de toutes les couches de l'ébauche céramique multicouche comprend du dioxyde de zirconium dopé à l'oxyde d'yttrium (Y2O3), à l'oxyde de calcium (CaO), à l'oxyde de magnésium (MgO) et/ou à l'oxyde de cérium (CeO2), dans lequel le dioxyde de zirconium est dopé à l'oxyde d'yttrium, le pourcentage d'oxyde d'yttrium dans les seconde, troisième et/ou autres couches étant compris entre 1 % en poids et 15 % en poids, de préférence entre 2 % en poids et 11 % en poids, et de façon encore préférentielle entre 2,5 % en poids et 10 % en poids.

14. Procédé selon l'une des précédentes revendications, **caractérisé en ce que** le matériau céramique de toutes les couches de l'ébauche céramique multicouche comprend du dioxyde de zirconium dopé à l'oxyde d'yttrium (Y2O3), à l'oxyde de calcium (CaO), à l'oxyde de magnésium (MgO) et/ou à l'oxyde de cérium (CeO2), dans lequel le dioxyde de zirconium est dopé à l'oxyde d'yttrium, le pourcentage d'oxyde d'yttrium dans la couche de couleur rose étant compris entre 0,3 % en poids et 10,5 % en poids, de préférence entre 0,6 % en poids et 7,5 % en poids, et de façon encore préférentielle entre 0,75 % en poids et 7 % en poids.
